Europäisches Patentamt

European Patent Office

Office européen des brevets

(19)

(11) Publication number: 0 159 087
A1

(12) EUROPEAN PATENT APPLICATION

(21) Application number: 85200533.9

(22) Date of filing: 04.04.85

(51) Int. Cl.⁴: **A 61 K 9/22**
A 61 L 17/00

(30) Priority: 04.04.84 NL 8401062

(43) Date of publication of application:
23.10.85 Bulletin 85/43

(84) Designated Contracting States:
AT BE CH DE FR GB IT LI LU NL SE

(71) Applicant: Stichting Biomaterials Science Center, VU,
"BSC-VU"
De Boelelaan 1115
NL-1081 HV Amsterdam(NL)

(72) Inventor: de Groot, Klaas
L. van Wijkplein 6
NL-2101 EL Heemstede(NL)

(74) Representative: Urbanus, Henricus Maria, Ir. et al,
c/o Vereenigde Octrooibureaux Nieuwe Parklaan 107
NL-2587 BP 's-Gravenhage(NL)

(54) A process for preparing an implant material which releases medicines in the body.

(57) Implant material for the release of medicine in bone
tissues or soft tissues, comprising a medicine and a carrier
composed of calcium sulphate and calcium carbonate.

EP 0 159 087 A1

A process for preparing an implant material which releases medicines in the body.

This invention relates to a system which after implantation releases a medicine to its surroundings, and which during the process of release is dissolved in full.

It can be inferred from an article by Wahling et al. (The release of gentamycin from polymethylmethacrylate beads, J. Bone Joint Surg. Br. Vol., 1978, 60, 270) that a two-component system consisting of beads of polymethylmethacrylate ("bone cement") in which the antibiotic gentamycin is dissolved is capable of successfully treating osteomyelitis. One disadvantage is that after the release of the antibiotic the beads of bone cement must be surgically removed from the bone. In the literature, therefore, certain methods are indicated of using, instead of the non-resorbing bone cement, a material that is dissolved in the body itself, so that surgical removal is unnecessary.

A first method is the use of beta-whitlockite instead of bone cement. Beta-whitlockite (chemical formula $Ca_3(PO_4)_2$) is, under certain conditions, more or less soluble. The biological dissolving process depends on the way in which the subject product has been sintered. (Sintering is a process - see a Chapter by K. de Groot in the book "Biocompatibility of Clinical Implant Materials", Editor D.F. Williams, C.R.C. Press, Boca Raton, USA, 1981, 199 - 224 - the principle of which is that by heating powder particles are cemented together via solid-state diffusion, so that from a powder, a solid body is formed). The rate of dissolution is promoted by ensuring that the powder particles are only connected together by means of small "necks". Eittenmuller et al. (see his paper entitled: Resorbierbare Antibiotik/Antiseptische TCP-keramik zur lokalen Behandlung der Osteomyelitis, 1.Koelner Workshop über Hydroxylkeramik, Cologne, December 7, 1983) have used this beta-whitlockite (also referred to as beta-TCP or β-TCP) in a highly porous form as a carrier for an antibiotic, instead of bone cement.

A second method is the use of calcium sulphate (Plaster of Paris). Varlet et al., in the article "Billes de platre de Paris aux antibiotique dans le traitement de l'infection osseuse", in "Revue de Chirurgie Orthopedique", 1983, 69, 239 - 244, have indicated and shown that gypsum, too, can function as a biologically soluble bead. Their method is predicated on gypsum being made up with the medicine to form a solid final product (by adding water), which like Eitenmuller's TCP has a highly porous composition. Other methods are not based on ceramic starting materials, and accordingly fall outside the scope of the present patent application.

Characteristic of both methods is that the medicine ("antibiotic") is released by diffusion, whereafter the remaining implant (the remaining component) is resorbed, so that the surgical removal of the beads is unnecessary. (It follows that the release of the medicine is not determined by the rate of biodegradation of the implant system).

Both methods, however, have certain disadvantages. Beta-TCP has the disadvantage that the dissolving process may have adverse side-effects. The cause is as follows. After being implanted, a porous beta-TCP product, on account of the loose bond between the sintered particles ("weak necks") disintegrates into loose particles, which in turn are carried off by macrophages to neighbouring lymph glands. Depending on the weakness of the bond between the sintered particles, the dissolving process which is thus effected is taking place at a faster or slower rate. (Non-porous beta-whitlockite is hardly resorbed). In certain cases the particles carried off are not intracellularly dissolved before the lymph glands are reached, so that, in that case, the lymph glands are filled with calcium phosphate particles. If no macrophages are present, or not enough, particles are accumulated around the implant, whereby further release is adversely affected. A second disadvantage of beta-TCP is that the dissolving process is biologically determined owing to the essential role of macrophages, so that there is no certainty as to the eventual disappearance of the implant.

Calcium sulphate, too, has a disadvantage: its rate of dissolution is so high that the implant is not always replaced by bone tissue, which may leave a void (Frame, J. Dent., 3, 177, 1975). One advantage, compared with beta-whitlockite, is that the dissolving process is a truly physico-chemical, and hence predictable one.

The present invention provides a (three-component) system which (1) unlike the beta-whitlockite described earlier, is dissolved not via disintegration into small particles, and hence without involving macro-phagical removal; and (2) is not dissolved as fast as gypsum, so that bone voids may be left. A third unique property (3) of the system is that the rate of decomposition of the implant is correlated to the rate of release of the medicine, so that a pre-programmed dosage is possible, in contrast to the earlier-described systems.

The invention is characterized by being a three-component system comprising a powdered gypsum product ($CaSO_4 - \frac{1}{2}H_2O$), a powdered calcium carbonate ($CaCO_3$), and a medicine. After mixing, the required amount of water is added (at least 20% of the gypsum content) whereby a dense final product is formed. Calcium carbonate has been chosen because, unlike gypsum (which, depending of course on the size of the implant, has disappeared in several weeks) requires several months for resorption (see, for example, Patel, in Revue du Palais de la Decouverte, 10, 18 - 23, 1981). Both calcium carbonate and gypsum are much more soluble than beta-TCP, so that both materials have a physico-chemical dissolving process without cellular interaction. To produce a good final product, at least 30% of gypsum is needed, otherwise it sets insufficiently when mixed with water. Variation of the gypsum-to-calcium carbonate ratio from 1.0 to 0.3 gives a variation in rate of decomposition from several weeks to several months. As the products are not porous, the release of medicine can be controlled by means of the rate of dissolution of the system. (There is no diffusion from pores, which do not exist).

- 4 -

C L A I M S
============

1.     Implant material for the release of a medicine in bone tissues or soft tissues, comprising a medicine and a carrier therefor, characterized in that the carrier is composed of calcium sulphate and calcium carbonate.

2.     Implant material as claimed in claim 1, characterized in that the carrier contains at least 30% by weight of calcium sulphate.

3.     An implant material as claimed in claim 1, characterized in that the carrier has a calcium sulphate-to-calcium carbonate ratio, by weight, of 0.3 to 1.0.

4.     A process for the production of an implant material for the release of medicine, comprising a medicine and a carrier therefor, characterized by mixing a medicine with calcium sulphate, or a hydrate thereof, and calcium carbonate, and setting the mixture by adding water.

5.     A process as claimed in claim 4, characterized by using at least 30% by weight of calcium sulphate, calculated on the mixture of calcium sulphate and calcium carbonate.

6.     A process as claimed in claim 4, characterized by using a calcium sulphate-to-calcium carbonate ratio, by weight, of 0.3 - 1.0.

7.     A process as claimed in claim 4, characterized by using at least 20% by weight of water, calculated on the amount of calcium sulphate.

## DOCUMENTS CONSIDERED TO BE RELEVANT

| Category | Citation of document with indication. where appropriate, of relevant passages | Relevant to claim | CLASSIFICATION OF THE APPLICATION (Int. Cl.4) |
|---|---|---|---|
| A | GB-A- 630 439 (ETHAN ALLAN BROWN) * Page 2, line 108 - page 3, line 34 * | 1-7 | A 61 K 9/22 A 61 L 17/00 |
| | --- | | |
| A | GB-A-2 093 348 (LEO PHARMACEUTICAL PROD. LTD.) * Page 4, lines 24-104 * | 1-7 | |
| | ----- | | |

TECHNICAL FIELDS
SEARCHED (Int. Cl.4)

A 61 K
A 61 L

The present search report has been drawn up for all claims

| Place of search THE HAGUE | Date of completion of the search 11-07-1985 | Examiner BRINKMANN C. |
|---|---|---|

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82